# FASCICULE DE BREVET EUROPEEN

(11) **EP 0 846 029 B1**
(45) Date de publication et mention de la délivrance du brevet: **12.01.2000**
(21) Numéro de dépôt: 96925817.7
(22) Date de dépôt: 18.07.1996
(51) Int. Cl.: B01J 31/40, B01J 38/60, C07C 51/42, C07C 57/03

(54) **PROCEDE DE SEPARATION D'UN CATALYSEUR AU PALLADIUM**
VERFAHREN ZUR TRENNUNG EINES PALLADIUMSKATALYSATOR
METHOD FOR SEPARATING A PALLADIUM CATALYST

(30) Priorité: 09.08.1995 FR 9509807
(43) Date de publication de la demande: 10.06.1998
(73) Titulaire: RHODIA FIBER & RESIN INTERMEDIATES, 92408 Courbevoie Cédex (FR)
(72) Inventeur: LECONTE, Philippe, F-69330 MEYZIEU (FR); PATOIS, Carl, F-68400 Riedisheim (FR)
(74) Mandataire: Vignally, Noel
(86) Numéro de dépôt international: FR9601130
(87) Numéro de publication internationale: WO9705949

(56) Documents cités:
- EP-A- 0 337 803
- EP-A- 0 546 324
- EP-A- 0 552 846
- US-A- 3 857 895
- US-A- 3 928 231

## Description

La présente invention concerne la séparation d'un catalyseur à base de palladium, à partir d'un milieu dans lequel il est dissous.

Plus spécifiquement, l'invention concerne la séparation d'un catalyseur à base de palladium, à partir d'un milieu provenant de la réaction d'hydroxycarbonylation du butadiène en acides penténoïques.

L'un des objets du procédé de l'invention réside dans la séparation d'au moins une partie du catalyseur au palladium dissous sous forme de complexe organométallique dans le milieu à traiter, afin de pouvoir recycler ce catalyseur au palladium dans une nouvelle réaction d'hydroxycarbonylation du butadiène.

Un deuxième objet du procédé est de permettre de séparer au moins une partie des acides penténoïques présents dans ledit milieu.

L'hydroxycarbonylation du butadiène et/ou de ses dérivés, tels que notamment les buténols allyliques comme le 3-butène-2-ol, le 2-butène-1-ol et leurs mélanges, les composés d'addition du chlorure d'hydrogène sur le butadiène (chlorobutènes) dont le principal est le chlorure de crotyle, peut être effectuée par l'eau et le monoxyde de carbone, sous une pression supérieure à la pression atmosphérique et en présence d'un catalyseur au palladium soluble dans le milieu réactionnel.

On peut se référer par exemple au brevet EP-A-0 648 731 pour une description plus détaillée d'une telle technique, bien que la présente invention ne soit pas limitée au traitement de mélanges réactionnels issus du procédé selon ce brevet.

Le brevet US-A-3 857 895 décrit un procédé d'hydroformylation d'une oléfine à l'aide de monoxyde de carbone et d'hydrogène en présence d'un catalyseur comprenant un complexe d'un métal du groupe VIII avec un ligand comportant au moins un groupe aminoalkyle ou amidinoalkyle ou aminoaryle ou amidinoaryle lié à un atome trivalent d'arsenic, d'antimoine ou de phosphore.

Le mélange issu de cette réaction est traité pour séparer le produit d'hydroformylation et le résidu contenant le catalyseur, ledit résidu est traité par une solution acide aqueux pour dissoudre le catalyseur, et la solution acide obtenue est séparée.

Dans ce procédé, le ligand réagit avec l'acide pour former des sels d'ammonium solubles dans la solution acide.

Les mélanges réactionnels mis en oeuvre dans le procédé de l'invention contiennent des quantités plus ou moins importantes des composés engagés dans la réaction d'hydroxycarbonylation et des composés formés lors de cette réaction.

Hormis le catalyseur au palladium qui peut se trouver sous diverses formes chimiques, le mélange réactionnel contient les acides penténoïques formés, notamment l'acide 3-penténoïque, de l'eau, de l'acide chlorhydrique, le plus souvent également des sous-produits de la réaction comme les butènes ou l'acide valérique, des diacides carboxyliques comme l'acide adipique, l'acide méthyl-2 glutarique, l'acide éthyl-2 succinique, éventuellement du butadiène n'ayant pas été transformé ainsi que l'éventuel solvant mis en oeuvre dans la réaction.

L'invention consiste donc en un procédé de séparation d'au moins une partie d'un catalyseur d'hydroxycarbonylation à base de palladium dissous dans une solution contenant aussi au moins de l'acide 3-penténoïque, caractérisé en ce que ladite solution est acidifiée et agitée avec une solution aqueuse d'acide chlorhydrique et en ce que l'on obtient deux phases liquides dont une phase aqueuse contenant au moins une partie du palladium

Lorsque la solution à traiter provient directement d'une opération d'hydroxycarbonylation du butadiène, il est évidemment nécessaire de supprimer la pression de monoxyde de carbone préalablement à l'acidification.

La solution aqueuse d'acide chlorhydrique utilisée contient généralement de 5 % à 40 % en poids d'acide chlorhydrique par poids de solution.

De manière générale, on ajoute la solution d'acide chlorhydrique à raison de 0,1 à 2 fois le volume de la solution à traiter.

La formation de deux phases liquides lors de l'acidification peut provenir de la simple addition de la solution aqueuse d'acide chlorhydrique selon la composition de la solution à traiter.

C'est notamment, mais pas exclusivement, le cas lorsque la solution à traiter contient un solvant essentiellement non-miscible à l'eau, tel qu'un hydrocarbure aromatique, aliphatique ou cycloaliphatique, un hydrocarbure aromatique chloré, aliphatique chloré ou cycloaliphatique chloré.

La séparation en deux phases liquides peut également être obtenue par l'ajout d'un solvant organique non-miscible à l'eau. Cet ajout peut être effectué après l'acidification, au moment de l'acidification ou le cas échéant avant l'acidification.

La présence d'un solvant organique non-miscible à l'eau permet de d'extraire au moins une partie des acides penténoïques présents dans la solution à traiter.

La température à laquelle est acidifiée la solution à traiter n'est pas réellement critique pour la mise en oeuvre du procédé. Ainsi peut-on opérer entre 0°C et 230°C (température à laquelle peut être conduite la réaction antérieure d'hydroxycarbonylation). En pratique, cependant, on opérera entre 20°C et 200°C et de préférence entre 40°C et 110°C.

L'acidification par l'acide chlorhydrique permet de transformer le palladium, présent sous forme de complexe organométallique dans la solution à traiter, en dihydrogénotétrachlorure de palladium. La température à laquelle on opère joue sur la vitesse de cette transformation des composés du palladium, une température plus élevée accélérant cette transformation, mais risquant de faire précipiter une partie du palladium.

Le solvant organique utilisé pour effectuer l'extraction est choisi avantageusement parmi les hydrocarbures aromatiques, aliphatiques ou cycloaliphatiques, les hydrocarbures aromatiques chlorés, aliphatiques chlorés ou cycloaliphatiques chlorés, liquides dans les conditions opératoires et essentiellement non-miscibles à l'eau.

Par commodité, le point d'ébullition du solvant organique sera inférieur à celui de l'acide 3-penténoïque.

A titre d'exemples non limitatifs de ces solvants, on peut citer le benzène, le toluène, les xylènes, les chlorobenzènes, le cyclohexane, le butadiène, les butènes, les alcanes tels que hexanes, heptanes, octanes, nonanes, décanes, undécanes, dodécanes, et les différents mélanges de plusieurs de ces solvants.

Le mélange, obtenu après acidification de la solution à traiter, et le cas échéant après addition du solvant organique, et agitation, décante au repos en une phase organique et une phase aqueuse.

Comme pour l'acidification, l'extraction par le solvant organique peut être effectuée à une température située entre 0°C et 230°C, plus fréquemment entre 20°C et 200°C et de préférence entre 40°C et 110°C.

La phase organique contient plus de la moitié de la quantité initiale d'acides penténoïques, l'essentiel du butadiène et des butènes et une partie des diacides carboxyliques éventuellement présents dans la solution à traiter.

La phase aqueuse contient plus de la moitié de la quantité de palladium ainsi qu'une partie des diacides carboxyliques éventuellement présents dans la solution à traiter.

L'opération d'extraction à l'aide d'un solvant organique peut être répétée plusieurs fois si on le souhaite.

Selon le solvant organique utilisé, la quantité d'acides penténoïques de la phase organique peut dépasser 60 % et même 75 % de la quantité initialement présente dans la solution à traiter. Les acides penténoïques, et plus particulièrement l'acide 3-penténoïque, peuvent ensuite être isolés de la phase organique par les moyens habituels de la chimie.

La phase aqueuse contient généralement plus de 60 % et de préférence plus de 80 % du palladium initialement présent dans la solution à traiter. Avec le procédé de l'invention, on peut même récupérer dans la phase aqueuse jusqu'à la quasi-totalité du palladium.

La phase aqueuse contenant le palladium peut avantageusement être recyclée dans une nouvelle réaction d'hydroxycarbonylation du butadiène. Généralement, il est souhaitable de distiller préalablement une partie de l'acide chlorhydrique qu'elle contient de manière à ajuster la quantité d'acide chlorhydrique à la quantité qu'il est opportun d'avoir pour l'hydroxycarbonylation. Par une telle distillation on obtient une solution d'acide chlorhydrique correspondant à l'azéotrope eau/chlorure d'hydrogène. Dans le cadre d'un procédé industriel en continu, il est particulièrement intéressant d'utiliser la solution d'acide chlorhydrique ainsi obtenue pour réaliser l'acidification de la solution initiale à traiter, après ajout du complément éventuellement nécessaire.

Une variante du procédé de l'invention consiste à distiller au moins une partie des acides penténoïques de la solution à traiter avant de réaliser l'acidification.

Une telle distillation sera effectuée à une température inférieure ou égale à 110°C. Cette limitation de la température est importante, car il a été observé que si l'on opère à une température plus élevée, une partie du palladium précipite. Une telle précipitation même partielle est inacceptable dans un procédé industriel. En effet, elle génère des pertes en un métal très coûteux et elle complique en outre sérieusement le traitement des mélanges réactionnels.

De manière inattendue, si l'on opère à une température inférieure ou égale à 110°C, et encore plus préférentiellement à une température inférieure ou égale à 105°C, on n'observe pas de précipitation de palladium.

Pour pouvoir respecter cette limite supérieure de température, il suffit parfois d'opérer sous pression atmosphérique. Il est plus souvent nécessaire de distiller les acides penténoïques sous une pression inférieure à la pression atmosphérique, généralement de l'ordre de 2 KPa à 7 KPa.

Avec les acides penténoïques distillent également les composés les plus légers pouvant être présents dans la solution à traiter, comme à titre d'exemples le butadiène, les butènes, l'eau, éventuellement une partie des diacides carboxyliques et le solvant éventuellement présent.

Le résidu obtenu après cette distillation contient le palladium ainsi que les composés les plus lourds, comme une autre partie des diacides carboxyliques. Ce résidu est ensuite traité comme décrit précédemment à l'aide d'une solution aqueuse d'acide chlorhydrique contenant généralement de 5 % à 40 % en poids d'acide chlorhydrique par poids de solution. Il n'est pas indispensable d'effectuer d'extraction à l'aide d'un solvant organique comme dans la première variante qui a été décrite.

Comme cela a été dit précédemment, la température à laquelle est acidifiée la solution à traiter peut se situer entre 0°C et 230°C, en pratique entre 20°C et 200°C et de préférence entre 40°C et 110°C.

La solution aqueuse acidifiée contenant le palladium peut être recyclée dans une nouvelle réaction d'hydroxycarbonylation du butadiène comme précédemment, le cas échéant après distillation de l'excès d'acide chlorhydrique.

Le palladium recyclé présente à quantité équivalente une activité catalytique semblable à celle que l'on observe avec un catalyseur neuf.

Outre le recyclage sous une forme homogène du catalyseur au palladium et l'isolement d'au moins une partie des acides penténoïques formés, le procédé de l'invention permet l'élimination d'une partie des sous-produits de la réaction d'hydroxycarbonylation du butadiène, notamment des diacides carboxyliques, dont l'accumulation peut s'avérer néfaste pour ladite réaction d'hydroxycarbonylation du butadiène.

Les exemples qui suivent illustrent l'invention.

### EXEMPLES 1 A 3

Dans un ballon agité magnétiquement sont placés successivement:
- 67 mg de PdCl₂
- 360 mg d'HCl à 37%
- 8 g d'acide 3-penténoïque (P3)
- 9 g d'acide méthyl-2 glutarique
- 3 g d'acide éthyl-2 succinique

On ajoute ensuite 20 ml d'HCl à 10 % on poids dans l'eau et 20 ml d'un solvant organique (indiqué dans le tableau 1 ci-après). On laisse agiter à température ambiante pendant 30 min. On sépare les deux phases par décantation et dose le palladium dans ces deux phases. Dans tous les cas, on n'observe pas de précipité. Les résultats des dosages sont rassemblés dans le tableau 1.

**Tableau 1**

| Exemples | solvant organique | Pd :% dans la phase aqueuse | Pd :% dans la phase organique |
|---|---|---|---|
| Exemple 1 | toluène | 100 | 0 |
| Exemple 2 | cyclohexane | 84 | 16 |
| Exemple 3 | dodécane | 80 | 20 |

### EXEMPLES 4 A 7

Dans un ballon agité magnétiquement sont placés successivement:
- 66,8 mg de π-crotyl-Pd-Cl
- 242 mg de chlorobutène
- 8,02 g d'acide 3-penténoïque (P3)
- 9,04 g d'acide méthyl-2 glutarique
- 3 g d'acide éthyl-2 succinique.

L'ensemble est porté à 50°C pour obtenir une solution homogène, puis ramené à une température T°C.

On ajoute alors 20 g d'une solution d'HCl dans l'eau (concentration C % en HCl indiquée dans le tableau 2) et 20 g de toluène.

Au bout d'une durée d'agitation variable selon les exemples, les deux phases sont séparées par décantation. Le palladium, ainsi que les produits organiques, sont dosés dans les deux phases.

Les coefficients de partage des divers produits sont les suivants (rapports des fractions massiques couche aqueuse/couche organique) sont rassemblés dans le tableau 2 ci-après.

**Tableau 2**

| Exemples | HCl C % en poids | T°C | Durée d'agitation | Pd | P3 | diacides |
|---|---|---|---|---|---|---|
| Exemple 4 | 10 | 40 | 90 min | > 1300 | 0,22 | 1,7 |
| Exemple 5 | 10 | 70 | 90 min | 440 | 0,28 | 2,0 |
| Exemple 6 | 20 | 40 | 60 min | 350 | 0,13 | 0,9 |
| Exemple 7 | 37 | 45 | 60 min | 700 | 0,16 | 0,9 |

### EXEMPLE 8

On répète l'exemple 6 en faisant en outre passer bulle à bulle 83 mmol de butadiène dans le mélange pendant l'extraction.

On obtient les coefficients de partage suivants :
- Pd = 36
- P3 = 0,13
- diacides = 0,9.

### EXEMPLE 9

Dans un ballon de 250 ml, sont chargés successivement :
- 1,668 g (9,4 mmol, soit 1 g de Pd) de PdCl₂
- 54,08 g d'HCl à 37 % en poids dans l'eau
- 4,07 g d'acide 3-penténoïque
- H₂O quantité suffisante pour 100 g de solution.

On distille à pression atmosphérique jusqu'à obtenir environ 70 ml de distillat (74,15 g). Ce distillat contient 3,2 g d'acide 3-penténoïque. Les vapeurs ont une température de 106-107°C et la température dans le ballon ne dépasse pas 110°C. On ne constate aucune précipitation de palladium.

### EXEMPLE 10

Dans un ballon monocol de 100 ml, est chargé un mélange réactionnel provenant d'une réaction d'hydroxycarbonylation du butadiène en présence de π-crotyl palladium-chlorure. Ce mélange a la composition suivante :
- 0,0707 g (0,357 mmol) de π-crotyl palladium-chlorure
- 0,2706 de chlorobutène (ou chlorure de crotyle)
- 8,0 g d'acide 3-penténoïque
- 9,1 g d'acide 2-méthylglutarique
   3,11 g d'acide 2-éthylsuccinique.

A ce mélange réactionnel sont ajoutés :
- 21 g de dichloroéthane
- 21 g d'une solution aqueuse d'acide chlorhydrique à 20 % en poids.

Le ballon surmonté d'un réfrigérant est placé dans un bain d'huile.

On maintient le mélange sous agitation à 40°C pendant 1h.

Après arrêt de l'agitation et décantation, on prélève dans chacune des deux phases liquides obtenues des échantillons pour doser le palladium.

On retrouve pratiquement tout le palladium présent dans le mélange de départ.

Le coefficient de partage massique couche aqueuse/couche organique est de 32.

## Revendications

1. Procédé de séparation d'au moins une partie d'un catalyseur d'hydroxycarbonylation à base de palladium dissous dans une solution contenant aussi au moins de l'acide 3-penténoïque, caractérisé en ce que ladite solution est acidifiée et agitée avec une solution aqueuse d'acide chlorhydrique et en ce que l'on obtient deux phases liquides dont une phase aqueuse contenant au moins une partie du palladium.

2. Procédé selon la revendication 1, caractérisé en ce que la solution aqueuse d'acide chlorhydrique utilisée contient de 5 % à 40 % en poids d'acide chlorhydrique par poids de solution.

3. Procédé selon l'une des revendications 1 ou 2, caractérisé en ce que la solution aqueuse d'acide chlorhydrique est ajoutée à raison de 0,2 à 2 fois le volume de la solution à traiter.

4. Procédé selon l'une des revendications 1 à 3, caractérisé en ce que la formation de deux phases liquides lors de l'acidification provient de la simple addition de la solution aqueuse d'acide chlorhydrique.

5. Procédé selon la revendication 4, caractérisé en ce que la solution à traiter contient un solvant essentiellement non-miscible à l'eau, tel qu'un hydrocarbure aromatique, aliphatique ou cycloaliphatique, un hydrocarbure aromatique chloré, aliphatique chloré ou cycloaliphatique chloré.

6. Procédé selon l'une des revendications 1 à 3, caractérisé en ce que la séparation en deux phases liquides est obtenue par l'ajout d'un solvant organique non-miscible à l'eau, ajout effectué après l'acidification, au moment de l'acidification ou le cas échéant avant l'acidification.

7. Procédé selon la revendication 6, caractérisé en ce que le solvant organique ajouté pour effectuer l'extraction est choisi parmi les hydrocarbures aromatiques, aliphatiques ou cycloaliphatiques, les hydrocarbures aromatiques chlorés, aliphatiques chlorés ou cycloaliphatiques chlorés, liquides dans les conditions opératoires et essentiellement non-miscibles à l'eau.

8. Procédé selon l'une des revendications 6 ou 7, caractérisé en ce que le solvant organique est choisi parmi le benzène, le toluène, les xylènes, les chlorobenzènes, le cyclohexane, le butadiène, les butènes, les alcanes tels que hexanes, heptanes, octanes, nonanes, décanes, undécanes, dodécanes, et les différents mélanges de plusieurs de ces solvants.

9. Procédé selon l'une des revendications 1 à 8, caractérisé en ce qu'il s'applique à la séparation d'un catalyseur à base de palladium, à partir d'un milieu provenant de la réaction d'hydroxycarbonylation du butadiène en acides penténoïques.

10. Procédé selon l'une des revendications 1 à 9, caractérisé en ce que les deux phases obtenues après décantation sont constituées d'une phase organique qui contient plus de la moitié de la quantité initiale d'acides penténoïques, l'essentiel du butadiène et des butènes et une partie des diacides carboxyliques éventuellement présents dans la solution à traiter, et d'une phase aqueuse qui contient plus de la moitié de la quantité de palladium ainsi qu'une partie des diacides carboxyliques éventuellement présents dans la solution à traiter.

11. Procédé selon l'une des revendications 1 à 10, caractérisé en ce que la phase aqueuse contenant le palladium est recyclée dans une nouvelle réaction d'hydroxycarbonylation du butadiène, de préférence après avoir distillé une partie de l'acide chlorhydrique qu'elle contient de manière à ajuster la quantité d'acide chlorhydrique à la quantité qu'il est opportun d'avoir pour l'hydroxycarbonylation.

12. Procédé selon la revendication 11, caractérisé en ce que la solution d'acide chlorhydrique, correspondant à l'azéotrope eau/chlorure d'hydrogène, ainsi obtenue est utilisée pour réaliser l'acidification de la solution initiale à traiter, après ajout du complément éventuellement nécessaire.

13. Procédé selon l'une des revendications 1 à 12, de séparation d'au moins une partie du palladium dissous dans une solution contenant aussi au moins de l'acide 3-penténoïque, caractérisé en ce que ledit procédé consiste à distiller au moins une partie des acides penténoïques de la solution à traiter avant de réaliser son acidification à l'aide d'une solution aqueuse d'acide chlorhydrique.

14. Procédé selon la revendication 13, caractérisé en ce que la distillation est effectuée à une température inférieure ou égale à 110°C et plus préférentiellement à une température inférieure ou égale à 105°C afin d'éviter la précipitation de palladium.

15. Procédé selon l'une des revendications 13 ou 14, caractérisé en ce que la distillation est conduite sous pression atmosphérique ou sous une pression inférieure à la pression atmosphérique, telle que située entre 2 KPa et 7 KPa.

16. Procédé selon l'une des revendications 13 à 15, caractérisé en ce que le résidu obtenu après distillation, contenant le palladium, est traité à l'aide d'une solution aqueuse d'acide chlorhydrique.

17. Procédé selon la revendication 16, caractérisé en ce que la solution aqueuse d'acide chlorhydrique utilisée contient de 5 % à 40 % en poids d'acide chlorhydrique par poids de résidu de distillation.

18. Procédé selon l'une des revendications 16 ou 17, caractérisé en ce que la solution aqueuse acidifiée contenant le palladium est recyclée dans une nouvelle réaction d'hydroxycarbonylation du butadiène, le cas échéant après distillation de l'excès d'acide chlorhydrique.

## Claims

1. Process for isolation of at least a proportion of a palladium-based hydroxycarbonylation catalyst dissolved in a solution also containing at least 3-pentenoic acid, characterized in that the said solution is acidified and stirred with an aqueous solution of hydrochloric acid, and in that two liquid phases are obtained, including an aqueous phase containing at least a proportion of the palladium.

2. Process according to Claim 1, characterized in that the aqueous solution of hydrochloric acid employed contains from 5 % to 40 % by weight of hydrochloric acid by weight of solution.

3. Process according to either of Claims 1 and 2, characterized in that the aqueous solution of hydrochloric acid is added in a proportion of 0.2 to 2 times the volume of the solution to be treated.

4. Process according to one of Claims 1 to 3, characterized in that the formation of two liquid phases during the acidification originates simply from the addition of the aqueous solution of hydrochloric acid.

5. Process according to Claim 4, characterized in that the solution to be treated contains an essentially water-immiscible solvent such as an aromatic, aliphatic or cycloaliphatic hydrocarbon or a chlorinated aromatic, chlorinated aliphatic or chlorinated cycloaliphatic hydrocarbon.

6. Process according to one of Claims 1 to 3, characterized in that the separation into two liquid phases is obtained by the addition of a water-immiscible organic solvent, an addition performed after the acidification, at the time of the acidification or, if appropriate, before the acidification.

7. Process according to Claim 6, characterized in that the organic solvent added in order to perform the extraction is chosen from aromatic, aliphatic or cycloaliphatic hydrocarbons and chlorinated aromatic, chlorinated aliphatic or chlorinated cycloaliphatic hydrocarbons which are liquid in the operating conditions and are essentially water-immiscible.

8. Process according to either of Claims 6 and 7, characterized in that the organic solvent is chosen from benzene, toluene, xylenes, chlorobenzenes, cyclohexane, butadiene, butenes, alkanes such as hexanes, heptanes, octanes, nonanes, decanes, undecanes and dodecanes and various mixtures of several of these solvents.

9. Process according to one of Claims 1 to 8, characterized in that it is applied to the isolation of a palladium-based catalyst from a mixture originating from the reaction of hydroxycarbonylation of butadiene to pentenoic acids.

10. Process according to one of Claims 1 to 9, characterized in that the two phases obtained after density separation consist of an organic phase which contains more than half of the initial quantity of pentenoic acids, most of the butadiene and of the butenes and a proportion of the dicarboxylic acids which may be present in the solution to be treated, and of an aqueous phase which contains more than half of the quantity of palladium and a proportion of the dicarboxylic acids which may be present in the solution to be treated.

11. Process according to one of Claims 1 to 10, characterized in that the aqueous phase containing the palladium is recycled into a new butadiene hydroxycarbonylation reaction, preferably after a proportion of the hydrochloric acid which it contains has been distilled off, so as to adjust the quantity of hydrochloric acid to the quantity that it is convenient to have for the hydroxycarbonylation.

12. Process according to Claim 11, characterized in that the hydrochloric acid solution, corresponding to the water/hydrogen chloride azeotrope, thus obtained is employed for acidifying the initial solution to be treated, after addition of the top-up which may be necessary.

13. Process, according to one of Claims 1 to 12, for isolation of at least a proportion of the palladium dissolved in a solution also containing at least 3-pencenoic acid, characterized in that the said process consists in distilling at least a proportion of the pentenoic acids from the solution to be treated before it is acidified with the aid of an aqueous solution of hydrochloric acid.

14. Process according to Claim 13, characterized in that the distillation is performed at a temperature which is lower than or equal to 110°C and more preferably at a temperature which is lower than or equal to 105°C in order to avoid the precipitation of palladium.

15. Process according to either of Claims 13 and 14, characterized in that the distillation is conducted at atmospheric pressure or at a pressure which is lower than atmospheric pressure, such as one situated between 2 kPa and 7 kPa.

16. Process according to one of Claims 13 to 15, characterized in that the residue obtained after distillation, containing the palladium, is treated with the aid of an aqueous solution of hydrochloric acid.

17. Process according to Claim 16, characterized in that the aqueous solution of hydrochloric acid employed contains from 5 % to 40 % by weight of hydrochloric acid per weight of distillation residue.

18. Process according to either of Claims 16 and 17, characterized in that the acidified aqueous solution containing the palladium is recycled into a new butadiene hydroxycarbonylation reaction, if appropriate after distillation of the excess of hydrochloric acid.

## Patentansprüche

1. Verfahren zur Abtrennung von wenigstens einem Teil eines Hydroxycarbonylierungskatalysators auf der Basis von Palladium, der in einer Lösung gelöst ist, die außerdem wenigstens 3-Pentensäure enthält, dadurch gekennzeichnet, daß besagte Lösung angesäuert und mit einer wassrigen Salzsäurelösung gerührt wird, und dadurch, daß man zwei flüssige Phasen erhält, deren eine wässrige Phase wenigstens einen Teil des Palladiums enthält.

2. Verfahren gemäß Anspruch 1, dadurch gekennzeichnet, daß die verwendete wässrige Salzsäurelösung 5 Gew.-% bis 40 Gew.-% Salzsäure pro Gewicht an Lösung enthält.

3. Verfahren gemäß einem der Ansprüche 1 oder 2, dadurch gekennzeichnet, daß die wässrige Salzsaurelösung in einer Menge des 0,2- bis 2-fachen des Volumens der zu behandelnden Lösung zugegeben wird.

4. Verfahren gemäß einem der Ansprüche 1 bis 3, dadurch gekennzeichnet, daß die Bildung von zwei flüssigen Phasen beim Ansäuern aus der einfachen Zugabe der wässrigen Salzsäurelösung herrührt.

5. Verfahren gemäß Anspruch 4, dadurch gekennzeichnet, daß die zu behandelnde Lösung ein im wesentlichen mit Wasser nicht mischbares Lösungsmittel, wie einen aromatischen, aliphatischen oder cycloaliphatischen Kohlenwasserstoff, einen chlorierten aromatischen, chlorierten aliphatischen oder chlorierten cycloaliphatischen Kohlenwasserstoff, enthält.

6. Verfahren gemäß einem der Ansprüche 1 bis 3, dadurch gekennzeichnet, daß die Trennung in zwei flüssige Phasen durch die Zugabe eines mit Wasser nicht mischbaren organischen Lösungsmittels erzielt wird, wobei die Zugabe nach dem Ansäuern, zum Zeitpunkt des Ansäuerns oder gegebenenfalls vor dem Ansäuern ausgeführt wird.

7. Verfahren gemäß Anspruch 6, dadurch gekennzeichnet, daß das organische Lösungsmittel, das zugegeben wird, um die Extraktion auszuführen, unter den aromatischen, aliphatischen oder cycloaliphatischen Kohlenwasserstoffen, den chlorierten aromatischen, chlorierten aliphatischen oder chlorierten cycloaliphatischen Kohlenwasserstoffen, die unter den Arbeitsbedingungen flüssig und im wesentlichen mit Wasser nicht mischbar sind, ausgewählt ist.

8. Verfahren gemäß einem der Ansprüche 6 oder 7, dadurch gekennzeichnet, daß das organische Lösungsmittel unter Benzen, Toluen, den Xylenen, den Chlorbenzenen, Cyclohexan, Butadien, den Butenen, den Alkanen, wie Hexanen, Heptanen, Octanen, Nonanen, Decanen, Undecanen, Dodecanen, und den verschiedenen Gemischen mehrerer dieser Lösungsmittel ausgewählt ist.

9. Verfahren gemäß einem der Ansprüche 1 bis 8, dadurch gekennzeichnet, daß es auf die Abtrennung eines Katalysators auf der Basis von Palladium, ausgehend von einem Medium, das aus der Hydroxycarbonylierungsreaktion des Butadiens zu Pentensäuren stammt, angewendet wird.

10. Verfahren gemäß einem der Ansprüche 1 bis 9, dadurch gekennzeichnet, daß die beiden nach dem Dekantieren erhaltenen Phasen aus einer organischen Phase, die mehr als die Hälfte der Anfangsmenge an Pentensäuren, die Hauptmenge des Butadiens und der Butene und einen Teil der gegebenenfalls in der zu behandelnden Lösung vorhandenen Dicarbonsäuren enthält, und aus einer wässrigen Phase, die mehr als die Hälfte der Palladiummenge sowie einen Teil der gegebenenfalls in der zu behandelnden Losung vorhandenen Dicarbonsäuren enthält, bestehen.

11. Verfahren gemäß einem der Ansprüche 1 bis 10, dadurch gekennzeichnet, daß die das Palladium enthaltende wässrige Phase in eine neue Hydroxycarbonylierungsreaktion des Butadiens zurückgeführt wird, vorzugsweise nachdem ein Teil der Salzsäure, die sie enthält, destilliert wurde, um die Menge an Salzsäure auf die Menge, die für die Hydroxycarbonylierung zweckmäßig ist, einzustellen.

12. Verfahren gemäß Anspruch 11, dadurch gekennzeichnet, daß die so erhaltene Salzsäurelösung, entsprechend dem Azeotrop Wasser/Chlorwasserstoff, nach Zugabe der gegebenenfalls notwendigen Ergänzungsmenge verwendet wird, um das Ansäuern der zu behandelnden Anfangslösung durchzuführen

13. Verfahren gemäß einem der Ansprüche 1 bis 12 zur Abtrennung von wenigstens einem Teil des Palladiums, das in einer Lösung gelöst ist, die außerdem wenigstens 3-Pentensäure enthält, dadurch gekennzeichnet, daß besagtes Verfahren darin besteht, wenigstens einen Teil der Pentensäuren der zu behandelnden Lösung zu destillieren, bevor man ihr Ansäuern mittels einer wässrigen Salzsäurelösung ausführt.

14. Verfahren gemäß Anspruch 13, dadurch gekennzeichnet, daß die Destillation bei einer Temperatur kleiner oder gleich 110°C und stärker bevorzugt bei einer Temperatur kleiner oder gleich 105°C ausgeführt wird, um das Ausfällen von Palladium zu vermeiden.

15. Verfahren gemäß einem der Ansprüche 13 oder 14, dadurch gekennzeichnet, daß die Destillation unter Atmosphärendruck oder unter einem Druck kleiner als Atmosphärendruck, wie zwischen 2 KPa und 7 KPa, betrieben wird.

16. Verfahren gemäß einem der Ansprüche 13 bis 15, dadurch gekennzeichnet, daß der nach Destillation erhaltene Rückstand, der das Palladium enthält, mittels einer wässrigen Salzsäurelösung behandelt wird.

17. Verfahren gemäß Anspruch 16, dadurch gekennzeichnet, daß die verwendete wässrige Salzsäurelösung 5 Gew.-% bis 40 Gew.-% Salzsäure pro Gewicht an Destillationsrückstand enthält.

18. Verfahren gemäß einem der Ansprüche 16 oder 17, dadurch gekennzeichnet, daß die das Palladium enthaltende angesäuerte wässrige Lösung in eine neue Hydroxycarbonylierungsreaktion des Butadiens zurückgeführt wird, gegebenenfalls nach Destillation des Salzsäureüberschusses.
